# EUROPEAN PATENT APPLICATION

(11) **EP 1 589 027 A1**
(43) Date of publication of application: **26.10.2005**
(21) Application number: 04700532.7
(22) Date of filing: 07.01.2004
(51) Int. Cl.: C07H 19/167, C07H 19/16, C07H 19/067, C07H 19/06

(54) **PROCESSES FOR PRODUCTION OF NUCLEOSIDES**

(30) Priority: 17.01.2003 JP 2003010373; 25.04.2003 JP 2003122614; 13.06.2003 JP 2003169534
(71) Applicant: Ajinomoto Co., Inc., Tokyo 104-8315 (JP)
(72) Inventor: TAKAHASHI, Daisuke, AJINOMOTO CO., INC., Kawasaki-shi, Kanagawa 210-8681 (JP); IZAWA, Kunisuke, AJINOMOTO CO., INC., Kawasaki-shi, Kanagawa 210-8681 (JP)
(74) Representative: Strehl Schübel-Hopf & Partner
(86) International application number: PCT/JP2004/000048
(87) International publication number: WO 2004/065403

(57) **Abstract**

The present invention relates to a production method of a nucleoside compound represented by the formula [II] which includes subjecting a 2',3',5'-triacyloxynucleoside compound represented by the formula [I] to deacylation using alkali metal hydroxide in a 0.01- to 0.5-fold amount in a molar ratio relative to the 2',3',5'-triacyloxynucleoside compound. According to the present invention, a production method of a nucleoside compound of the formula [II] which suppresses a by-product, and a production method of a nucleoside derivative which utilizes this method can be provided. In addition, the present invention relates to oxidation of a nucleoside compound represented by the formula (1) in the presence of a 2,2,6,6-tetramethylpiperidine-1-oxy catalyst, and hypochlorite or hypobromite, while adjusting pH to fall within the range of 5-9, and further, extracting a nucleoside-carboxylic acid compound represented by the formula (2) into an organic solvent under acidic conditions, back-extracting the compound from the organic solvent into an aqueous alkali solution, and neutralizing the aqueous alkali solution by adding an acid thereto to allow precipitation of a crystal. Thus, a highly pure particular nucleoside-carboxylic acid compound or a salt thereof can be produced by a method suitable for industrial production: 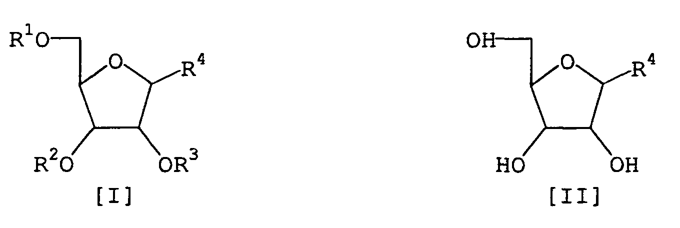 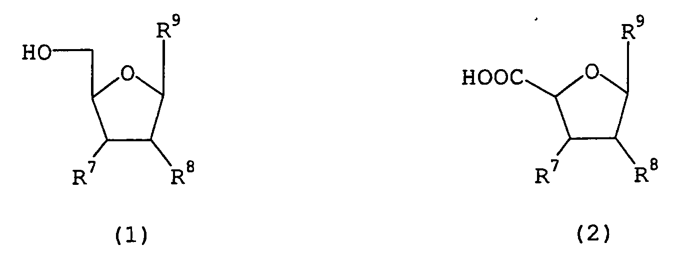 wherein each symbol is as defined in the Description.

## Description

### Technical Field

The present invention relates to a production method of a particular nucleoside compound and a derivative thereof.

Moreover, the present invention relates to a production method of a nucleoside-carboxylic acid compound or a salt thereof, more particularly, a production method of a nucleoside-carboxylic acid compound or a salt thereof, which comprises oxidation of a nucleoside compound under particular conditions, and further a production method of a crystal of a nucleoside-carboxylic acid compound or a salt thereof, which comprises crystal precipitation of the compound or a salt thereof under particular conditions, and the like.

### Background Art

A nucleoside compound represented by the formula [II]: 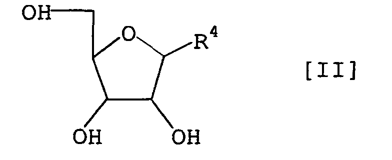 wherein R⁴ is a group represented by 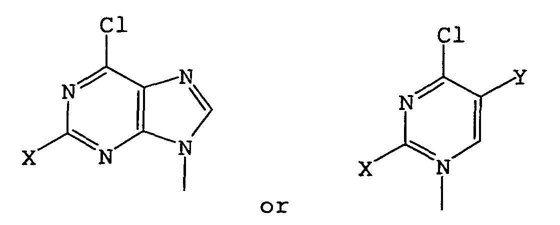 wherein X is a hydrogen atom, a halogen atom, an amino group, an alkyl group, an aralkyl group, a substituted amino group or a hydroxyl group, and Y is a hydrogen atom, a halogen atom, an alkyl group, an aralkyl group or an aryl group is useful as a synthetic intermediate for pharmaceutical products. There are various known production methods for the nucleoside compound. For example, conventional methods include subjecting a sugar compound, wherein three hydroxyl groups of a ribose skeleton have been protected, to a coupling reaction with a nucleic acid base, or subjecting nucleoside, after protecting three hydroxyl groups of the sugar moiety thereof, to various reactions for modifying a nucleic acid base, thereby producing a hydroxyl-protected nucleoside compound represented by the following formula: 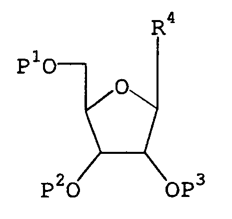 wherein R⁴ is as defined above and P¹, P² and P³ are the same or different and each independently is a hydroxyl-protecting group. (hereinafter sometimes to be abbreviated as a protected compound), which is followed by deprotection.

As specific examples of deprotection of a protected compound, wherein a nucleic acid base is a purine base, and a compound analogous thereto, the methods shown in the following scheme can be mentioned. 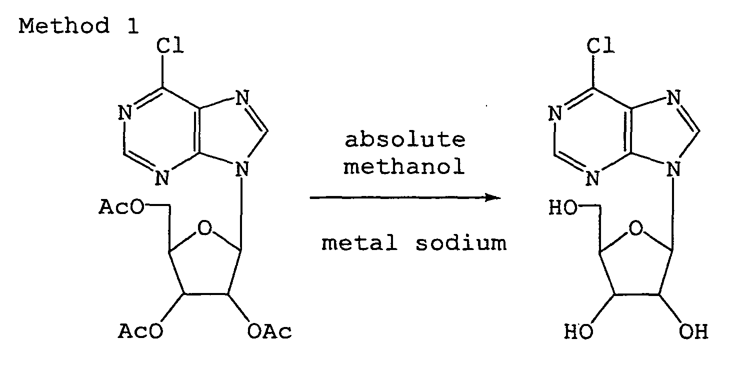 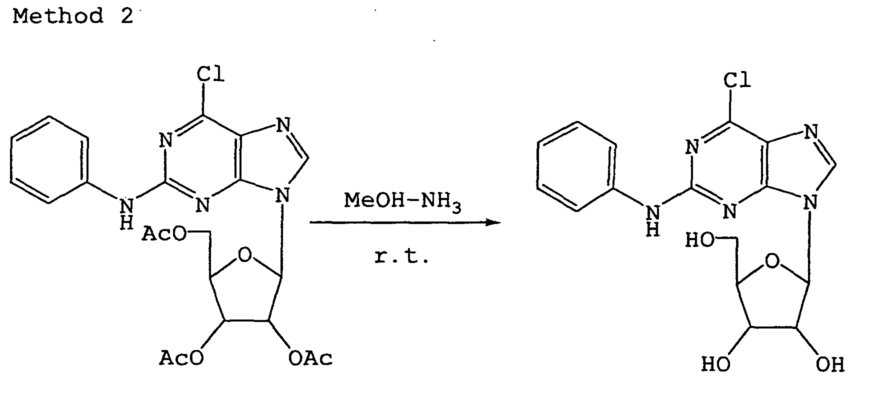
Method 1: In an ice bath, metal sodium is added three times every 1 hour to a protected compound dissolved in absolute methanol and the mixture is stirred (e.g., Journal of Medicinal Chemistry, (US), 1985, vol. 28, No. 11, p. 1642).
Method 2: A protected compound is reacted with methanolic ammonia at room temperature (e.g., Bharat K Trivedi et al., STUDIES TOWARD SYNTHESIS OF C2-SUBSTITUTED ADENOSINES: AN EFFICIENT SYNTHESIS OF 2-(PHENYLAMINO)ADENOSINE), NUCLEOSIDES & NUCLEOTIDES, (US), Marcel Dekker Inc., 1988, vol. 7, No. 3, pp. 393-402).

Method 1 is associated with a problem in that a by-product (6-methoxy form), wherein a chlorine atom at the 6-position is substituted by a methoxy group, is produced, and Method 2 is also associated with a problem in that ammonia (NH₃) is reacted at the position of a chlorine atom to give a by-product (6-amino form).

Various reports have been made on the deprotection of a protected compound wherein the 6-position of the purine ring of the protected compound is not a chloro group but an oxo group and, for example, a method using an aqueous potassium hydroxide solution and the like in not less than an equivalent amount relative to a hydroxyl-protecting group (e.g., acetyl group and the like) in an aqueous methanol solution (e.g., Journal of Organic Chemistry, (US), 1988, vol. 53, No. 3, p. 505), and the like can be mentioned. However, when this method is applied to a 6-chloro-2',3',5'-hydroxyl-protected-nucleoside compound, hydrolysis occurs at the 6-position (chloro-position) to give a by-product (6-hydroxyl form). In addition, since an aqueous organic solvent is used in the above-mentioned method, when a protected compound is subjected to a deacetylation reaction with alkali metal hydroxide, the released protecting group becomes an acid (acetic acid when the protecting group is an acetyl group), which is further neutralized with alkali metal hydroxide used for deacetylation, whereby a salt is by-produced in an almost stoichiometric amount relative to the protected compound. Therefore, a step for removing the salt by-produced in the above-mentioned method by extraction, washing and the like becomes necessary.

Thus, the development of a production method of a nucleoside compound, which suppresses a side reaction (e.g., reaction at the chloro group of the 6-position when nucleic acid base is a purine base, reaction at the chloro group of the 4-position in the case of a pyrimidine base and the like) and production of a salt and which, in consequence, can suppress by-production, has been desired.

In addition, it is known that a nucleoside-carboxylic acid compound represented by 2',3'-isopropylidene-6-chloropurineriboside-5'-carboxylic acid represented by the following formula (6) 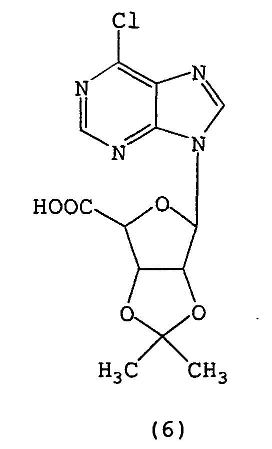 is useful as a synthetic intermediate for a pharmaceutical product and the like. As a production method of such nucleoside-carboxylic acid compound, for example, a method comprising oxidizing the corresponding 5'-hydroxyl group of a nucleoside compound to lead to a 5'-carboxyl group is known. For example, in JP-A-2000-514801, Example 1, a method comprising oxidizing 2',3'-isopropylidene-6-chloropurineriboside with sodium bromite in the presence of 4-hydroxy-2,2,6,6-tetramethylpiperidine-1-oxybenzoate to give 2',3'-isopropylidene-6-chloropurineriboside-5'-carboxylic acid is disclosed. Furthermore, 2',3'-isopropylidene-6-chloropurineriboside-5'-carboxylic acid is isolated as a solid from the obtained reaction mixture by extraction, concentration and vacuum drying.

Sodium bromite used in the above-mentioned known method is a reagent having high reactivity and use thereof on an industrial scale is not necessarily appropriate from the aspects of control of reaction and safety. Therefore, the present inventors considered use of hypochlorite or hypobromite, which are milder oxidants, and studied further.

When hypochlorite or hypobromite is used as an oxidant, however, the reaction mixture tends to have a high pH value. As a result of the consideration by the present inventors, the tendency toward formation of a hydroxyl group due to hydrolysis of a chloro group of the nucleoside compound has been found when the oxidation is carried out while the pH of the reaction mixture is high. Particularly, these commercially available oxidants show high basicity of pH 12 or above in an aqueous solution state, and the tendency toward hydrolysis is remarkable. The impurity (hydroxyl derivative) generated by hydrolysis during the oxidation cannot be removed easily in subsequent steps and causes a serious problem in terms of quality.

In the above-mentioned patent reference, moreover, a nucleoside-carboxylic acid compound is extracted from the reaction mixture into an organic solvent, and concentrated to dryness to give a nucleoside-carboxylic acid compound as a solid. While the present inventors tried crystal precipitation of a nucleoside-carboxylic acid compound from such organic solvent in an attempt to increase purification efficiency, it was found that the crystallinity of the compound was poor as evidenced by the production of the object product as an oil and the like, and the impurity generated by the above-mentioned hydrolysis could not be removed efficiently.

### Disclosure of the Invention

An object of the present invention is to provide (1) a production method of a nucleoside compound that suppresses a by-product. Another object of the present invention is to provide (2) a production method of a nucleoside derivative, which utilizes the production method of a nucleoside compound. A further object of the present invention is to provide (3) a production method of a nucleoside-carboxylic acid compound or a salt thereof, which is suitable for industrial production, namely, to provide an efficient production method of a nuecleoside-carboxlic acid compound wherein hydrolysis, which is a side reaction of an oxidation, is suppressed in a production system which uses, in a production process of a nucleoside-carboxylic acid compound, which is a 5'-carboxyl group derivative, or a salt thereof, comprising oxidation of a 5'-hydroxyl group of the nucleoside compound, hypochlorite or hypobromite, which is highly safe and permits easy control of the reaction, as an oxidant. A still further object of the present invention is to provide (4) a production method of a crystal of a nucleoside-carboxylic acid compound, which can efficiently remove hydrolysate produced during an oxidation.

The present inventors have conducted intensive studies in an attempt to solve the aforementioned problems. As a result, they have found that a nucleoside compound can be produced while suppressing a by-product, by subjecting a 2',3',5'-triacyloxynucleoside compound represented by the formula [I] to be mentioned below to deacylation using alkali metal hydroxide in a 0.01- to 0.5-fold amount in a molar ratio relative to the 2',3',5'-triacyloxynucleoside compound.

The present inventors have also found that the production of hydrolysate can be markedly suppressed by carrying out an oxidation of a nucleoside-carboxylic acid compound while adjusting the pH value during the oxidation to fall within a particular range. Furthermore, the present inventors have found that a highly pure crystal of a nucleoside-carboxylic acid compound, wherein the impurity generated due to hydrolysis during an oxidation has been markedly reduced, can be obtained by extracting a nucleoside-carboxylic acid compound in a reaction mixture into an organic solvent under acidic conditions, back-extracting the compound from the organic solvent into an aqueous alkali solution, and neutralizing the aqueous alkali solution by adding an acid thereto to allow precipitation of a crystal.

The present inventors have completed the present invention based on these findings.

Accordingly, the present invention provides the following.
[1] A production method of a nucleoside compound represented by the formula [II]: 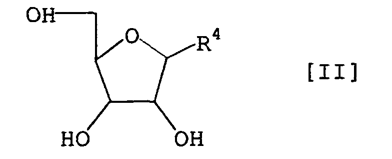 wherein R⁴ is a group represented by 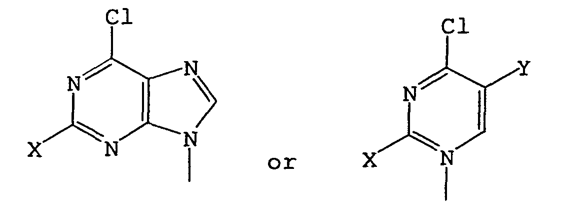 wherein X is a hydrogen atom, a halogen atom, an amino group, an alkyl group, an aralkyl group, a substituted amino group or a hydroxyl group, and Y is a hydrogen atom, a halogen atom, an alkyl group, an aralkyl group or an aryl group
   (hereinafter sometimes to be simply abbreviated as nucleoside compound [II]), which comprises
   subjecting a 2',3',5'-triacyloxynucleoside compound represented by the formula [I]: 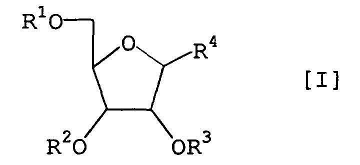 wherein R¹, R² and R³ are the same or different and each independently is an acyl group, and R⁴ is as defined above (hereinafter sometimes to be simply abbreviated as a 2',3',5'-triacyloxynucleoside compound) to deacylation using alkali metal hydroxide in a 0.01- to 0.5-fold amount in a molar ratio relative to the 2',3',5'-triacyloxynucleoside compound.
[2] The production method of the above-mentioned [1], wherein the deacylation is conducted in methanol, or a mixed solvent of methanol and an organic solvent.
[3] The production method of the above-mentioned [1] or [2], wherein the alkali metal hydroxide is sodium hydroxide.
[4] A production method of a 2',3'-hydroxyl-protected nucleoside compound represented by the formula [III]: 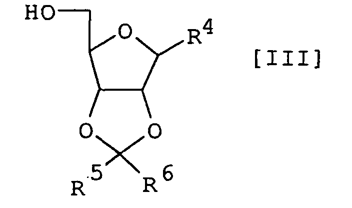 wherein R⁴ is as defined in the above-mentioned [1], and R⁵ and R⁶ are optionally the same or different and each independently is an alkyl group
   (hereinafter sometimes to be simply abbreviated as a 2',3'-hydroxyl-protected nucleoside compound), which comprises a step of obtaining a nucleoside compound represented by the formula [II] by the production method of any of the above-mentioned [1]-[3].
[5] A production method of a carboxylic acid compound represented by the formula [IV]: 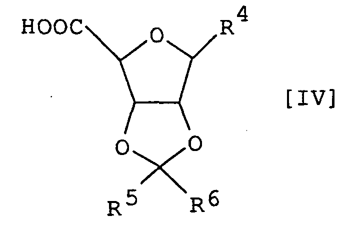 wherein R⁴ is as defined in the above-mentioned [1], and R⁵ and R⁶ are optionally the same or different and each independently is an alkyl group
   (hereinafter sometimes to be simply abbreviated as a carboxylic acid compound), which comprises a step of obtaining a nucleoside compound represented by the formula [II] by the production method of any of the above-mentioned [1]-[3].
[6] The production method of the above-mentioned [5], wherein the nucleoside compound of the formula [II] is converted to a 2',3'-hydroxyl-protected nucleoside compound of the formula [III], which is subsequently oxidized to give the carboxylic acid compound of the formula [IV].
[7] A production method of a nucleoside-carboxylic acid compound represented by the formula (2) (hereinafter sometimes to be simply abbreviated as a nucleoside-carboxylic acid compound) or a salt thereof, which comprises oxidizing a nucleoside compound represented by the formula (1) (hereinafter sometimes to be simply abbreviated as nucleoside compound (1)) in the presence of a 2,2,6,6-tetramethylpiperidine-1-oxy catalyst, and hypochlorite or hypobromite, while adjusting pH to fall within the range of 5-9: 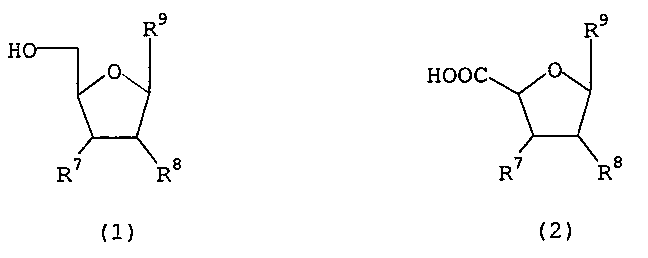 wherein R⁹ is a group represented by the following formula (3) or (4), and R⁷ and R⁸ are each independently a hydrogen atom, an acyloxy group, an alkyloxy group, an aralkyloxy group or a *tert*-butyldimethylsilyloxy group, or R⁷ and R⁸ in combination show a group of the following formula (5): 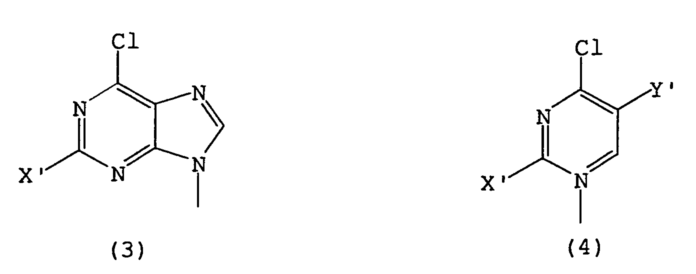 wherein X' is a hydrogen atom, a halogen atom, an amino group, a substituted amino group or a hydroxyl group, and Y' is a hydrogen atom, a halogen atom, an alkyl group or an aralkyl group: 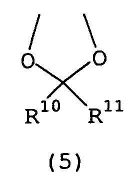 wherein R¹⁰ and R¹¹ are each independently an alkyl group.
[8] The production method of the above-mentioned [7], which comprises a step of decomposing the oxidant remaining in the reaction mixture with hydrogen sulfite after the completion of the oxidation.
[9] The production method of the above-mentioned [7], which comprises a step of producing a crystal of a nucleoside-carboxylic acid compound represented by the formula (2) by extracting the nucleoside-carboxylic acid compound in a reaction mixture into an organic solvent under acidic conditions, back-extracting the compound from the organic solvent into an aqueous alkali solution, and neutralizing the aqueous alkali solution by adding an acid thereto to allow precipitation of a crystal.
[10] The production method of the above-mentioned [7], which comprises a step of producing a crystal of a salt of the nucleoside-carboxylic acid compound represented by the formula (2) by precipitating a crystal after extracting the nucleoside-carboxylic acid compound in a reaction mixture into an organic solvent under acidic conditions, and back-extracting the compound from the organic solvent into an aqueous alkali solution to allow neutralization or back-extracting the compound from the organic solvent into water and neutralization with an aqueous alkali solution.
[11] A production method of a crystal of a nucleoside-carboxylic acid compound represented by the formula (2), which comprises extracting the nucleoside-carboxylic acid compound represented by the formula (2) into an organic solvent under acidic conditions, back-extracting the compound from the organic solvent into an aqueous alkali solution, and neutralizing the aqueous alkali solution by adding an acid thereto to allow crystal precipitation of the nucleoside-carboxylic acid compound: 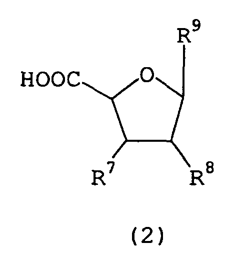 wherein R⁹ is a group represented by the following formula (3) or (4), and R⁷ and R⁸ are each independently a hydrogen atom, an acyloxy group, an alkyloxy group, an aralkyloxy group or a *tert*-butyldimethylsilyloxy group, or R⁷ and R⁸ in combination show a group represented by the following formula (5): 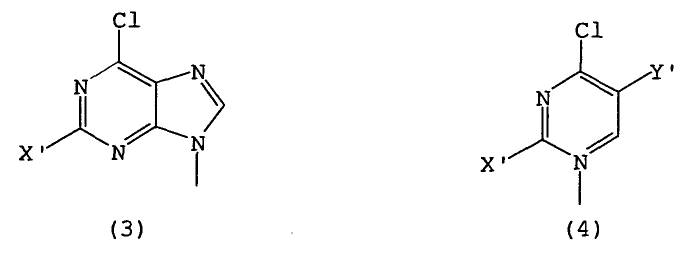 wherein X' is a hydrogen atom, a halogen atom, an amino group, a substituted amino group or a hydroxyl group, and Y' is a hydrogen atom, a halogen atom, an alkyl group or an aralkyl group, 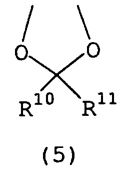 wherein R¹⁰ and R¹¹ are each independently an alkyl group.
[12] The production method of any of the above-mentioned [7]-[11], wherein the nucleoside-carboxylic acid compound represented by the formula (2) is a 2',3'-isopropylidene-6-chloropurineriboside-5'-carboxylic acid represented by the following formula (6):

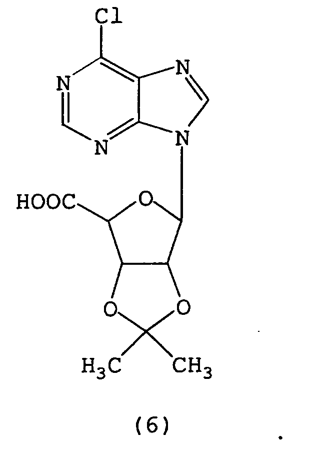

### Detailed Description of the Invention

The present invention is explained in detail by referring to the following reaction scheme.

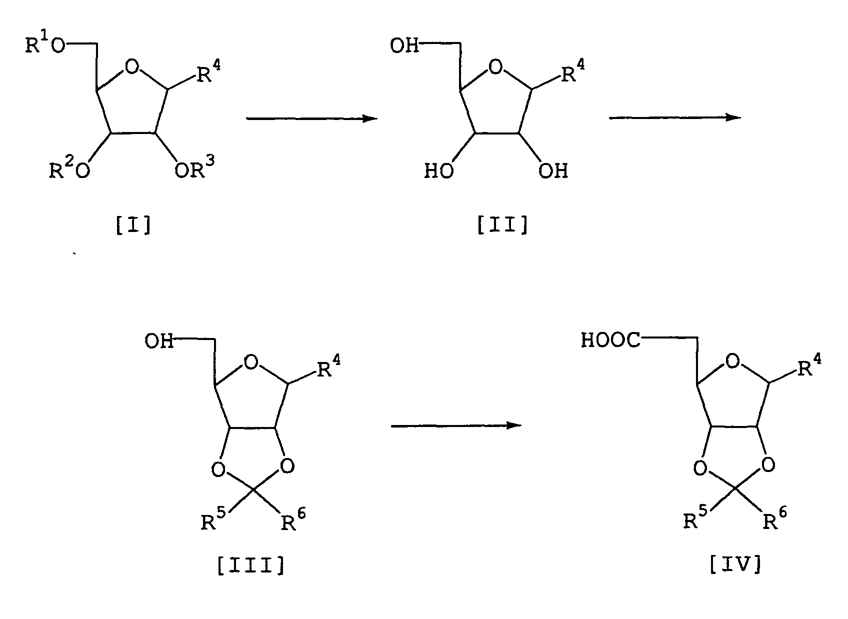

Each symbol in the formula is as defined in the following.

R¹, R² and R³ in the present invention may be the same or different and each independently is an acyl group. The acyl group here is an acyl group generally having 1 to 20, preferably 2 to 8, carbon atoms and, for example, acetyl, propionyl, benzoyl and the like can be mentioned, with preference given to acetyl.

X in the present invention is a hydrogen atom, a halogen atom, an amino group, an alkyl group, an aralkyl group, a substituted amino group or a hydroxyl group, and Y is a hydrogen atom, a halogen atom, an alkyl group, an aralkyl group or an aryl group.

The halogen atom here is a chlorine atom, a fluorine atom, a bromine atom or an iodine atom, with preference given to a chlorine atom.

The alkyl group here is a linear or branched chain alkyl group preferably having 1 to 10, more preferably 1 to 3, carbon atoms. For example, methyl, ethyl, propyl and the like can be mentioned, with preference given to methyl.

The aralkyl group here is an aralkyl group wherein the alkyl moiety is a linear or branched chain preferably having 1 to 5, more preferably 1, carbon atom, and the aryl moiety preferably has 6 to 10, more preferably 6 to 8, carbon atoms. As preferable examples, benzyl and the like can be mentioned.

The aryl group here preferably has 6 to 10, more preferably 6 to 8. As preferable examples, a phenyl group and the like can be mentioned.

The substituted amino group is an amino group monosubstituted or di-substituted by the following substituent and the like. The di-substituted amino group may have the same or different substituents. As the substituent, for example, an acyl group (as defined above, preferably having 1 to 7 carbon atoms, for example, acetyl, propionyl, benzoyl and the like can be mentioned, and acetyl is particularly preferable), an alkyl group (as defined above, and methyl and ethyl are particularly preferable), an aryl group (as defined above, and phenyl is particularly preferable), an aralkyl group (as defined above, and benzyl is particularly preferable) and the like can be mentioned. As the substituted amino group, acetylamino, methylamino, ethylamino, phenylamino, benzylamino and the like can be mentioned, with preference given to acetylamino and benzylamino.

R⁵ and R⁶ in the present invention are optionally the same or different and each independently is an alkyl group. The alkyl group here is defined to be the same as the alkyl group for X or Y.

### Production method of nucleoside compound [II]

The production method of nucleoside compound [II] in the present invention is characterized by deacylation of a 2',3',5'-triacyloxynucleoside compound using alkali metal hydroxide in a 0.01- to 0.5-fold amount in a molar ratio relative to the 2',3',5'-triacyloxynucleoside compound. To be specific, for example, a 2',3',5'-triacyloxynucleoside compound and alkali metal hydroxide (0.01-0.5 mol of the 2',3',5'-triacyloxynucleoside compound) are added to a solvent and the mixture is stirred.

As the solvent to be used for the production of nucleoside compound [II], methanol-containing solvents (e.g., methanol, or a mixed solvent of methanol and an organic solvent and the like) can be mentioned. Here, as the methanol-containing solvent, a solvent containing methanol generally at not less than 10%, preferably not less than 50%, more preferably not less than 70%, by volume can be used. The solvent contained other than methanol is not particularly limited as long as it is an organic solvent and, for example, tetrahydrofuran, acetonitrile and the like can be mentioned. The solvent usable for the production of nucleoside compound [II] is particularly preferably a single solvent of methanol. When the methanol-containing solvent contains a considerable amount of water, a by-product tends to occur. From the aspects of improvement of yield and the like, therefore, it is preferable to not use water. However, if the amount of water in the methanol-containing solvent does not cause substantial influence on the reaction, water may be contained in the solvent. The total amount of the solvent to be used is generally 2-20, preferably 5-15, relative to the 2',3',5'-triacyloxynucleoside compound in a weight ratio.

As the alkali metal hydroxide, for example, sodium hydroxide, potassium hydroxide, lithium hydroxide and the like can be mentioned, with preference given to sodium hydroxide. The amount of alkali metal hydroxide to be used in the present invention is essentially 0.01- to 0.5-fold of the 2',3',5'-triacyloxynucleoside compound in a molar ratio. When the amount of alkali metal hydroxide to be used exceeds this range, production of a by-product (6-methoxy form when nucleic acid base is purine base, 4-methoxy form in the case of pyrimidine base) increases and when the amount is smaller, the reaction does not complete. A preferable amount of use is 0.03- to 0.4-fold of the 2',3',5'-triacyloxynucleoside compound in a molar ratio.

The temperature for the production of nucleoside compound [II] is generally -20°C to 50°C, preferably 0°C to 20°C.

After the completion of the reaction, nucleoside compound [II] can be isolated and purified by conventional methods. For example, after the completion of the reaction, an acid (e.g., acetic acid and the like) and an organic solvent (e.g., ethyl acetate and the like) are added, the mixture is stirred, and the precipitate is collected by filtration and dried to give nucleoside compound [II].

### Production method of 2',3'-hydroxyl-protected nucleoside compound

The 2',3'-hydroxyl-protected nucleoside compound in the present invention can be easily produced from nucleoside compound [II] in the present invention by a method known to those of ordinary skill in the art. As the 2',3'-hydroxyl-protected nucleoside compound, an isopropylidene compound represented by the following formula (V): 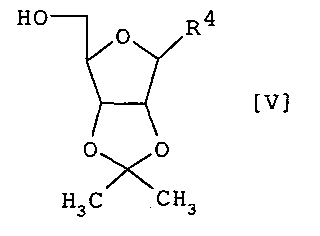 wherein R⁴ is as defined above
is particularly preferable. For example, nucleoside compound [II] obtained by the method of the present invention is converted to an isopropylidene compound of the formula [V] by a conventional method by, for example, (A) reacting nucleoside compound [II] with 2,2-dimethoxypropane in an aprotic organic solvent in the presence of an acid catalyst, or (B) reacting nucleoside compound [II] with acetone to be used as a solvent and reaction reagent in the presence of an acid catalyst.

As the acid catalyst to be used in the above-mentioned methods (A) and (B), for example, inorganic acids such as hydrochloric acid, sulfuric acid and the like, and organic acids such as methanesulfonic acid, *p*-toluenesulfonic acid and the like (including hydrates thereof) can be mentioned. The amount of the acid catalyst to be used is generally 0.01 to 1-fold, preferably 0.01 to 0.5-fold, in a molar ratio relative to nucleoside compound [II]. As the aprotic organic solvent to be used in the above-mentioned method (A), for example, acetone, acetonitrile, tetrahydrofuran, dichloromethane and the like can be mentioned, and the amount thereof to be used is generally 5-50, preferably 8-20, in a weight ratio relative to nucleoside compound [II].

The amount of 2,2-dimethoxypropane to be used in the above-mentioned method (A) is generally 1-5, preferably 1-3, in a molar ratio relative to nucleoside compound [II].

The amount of acetone to be used in the above-mentioned method (B) is generally 5-50, preferably 8-20, in a weight ratio relative to nucleoside compound [II].

In the above-mentioned (A) and (B), conversion of nucleoside compound [II] to an isopropylidene compound is performed at a reaction temperature of generally 0°C-50°C, preferably 0°C-room temperature, and the reaction completes generally in 0.5-24 hr, preferably 1-5 hr.

2',3'-Hydroxyl-protected nucleoside compounds other than the isopropylidene compound can be also produced by a method similar to the above-mentioned production method of the isopropylidene compound or a method analogous thereto.

The 2',3'-hydroxyl-protected nucleoside compound can be isolated and purified by a conventional method and, for example, the reaction mixture is added to an aqueous alkali (e.g., sodium hydrogen carbonate etc.) solution, the mixture is concentrated under reduced pressure, and the precipitate is filtered, washed with water and dried.

### Production method of carboxylic acid compound

The carboxylic acid compound in the present invention can be produced from nucleoside compound [II] in the present invention via, for example, the 2',3'-hydroxyl-protected nucleoside compound in the present invention. The 2',3'-hydroxyl-protected nucleoside compound is particularly preferably an isopropylidene compound represented by the aforementioned formula [V] and the carboxylic acid compound is particularly preferably an isopropylidenecarboxylic acid compound represented by the following formula [VI]: 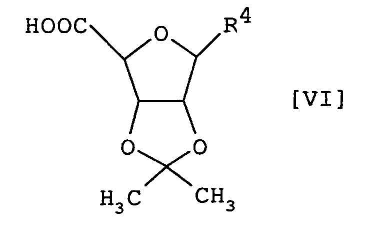 wherein R⁴ is as defined above.

The 2',3'-hydroxyl-protected nucleoside compound can be converted to a carboxylic acid compound by oxidation of a 2',3'-hydroxyl-protected nucleoside compound. As such method, conventionally known methods, for example, the methods described in JP-A-2000-524801, Tetrahedron Letters, vol. 37, No. 10, 1567-1570 and the like, methods analogous thereto and the like can be mentioned.

As a method capable of producing a carboxylic acid compound at a higher purity than by these conventionally known methods, a method comprising oxidation of a 2',3'-hydroxyl-protected nucleoside compound in the presence of a 2,2,6,6-tetramethylpiperidine-1-oxy catalyst (TEMPO catalyst), and hypochlorite or hypobromite, while adjusting pH to fall within the range of 5 to 9, can be mentioned.

As a reaction solvent usable for the above-mentioned reaction, a mixed solvent of water and an organic solvent, and a two-phase solvent wherein an aqueous phase and an organic solvent phase are phase-separated are preferable. The organic solvent may be any as long as it is free of an influence of an oxidation and, for example, for use for a mixed solvent with water, acetonitrile, tetrahydrofuran, acetone and the like can be mentioned, and for use for a two-phase solvent, chloroform, dichloromethane, *tert*-butylmethylether, ethyl acetate and the like can be mentioned. The amount of the reaction solvent to be used is generally 3-50, preferably 5-20, in a weight ratio relative to a 2',3'-hydroxyl-protected nucleoside compound. When a mixed solvent of water and an organic solvent or a two-phase solvent is used as a reaction solvent, the total amount of the solvent only needs to be included in this range.

As the TEMPO catalyst, TEMPO-like compounds showing an oxidation catalytic function similar to that of TEMPO can be mentioned besides TEMPO. As the TEMPO-like compound, for example, 4-hydroxy-2,2,6,6-tetramethylpiperidin-1-oxy, 4-methoxy-2,2,6,6-tetramethylpiperidin-1-oxy, 4-hydroxy-2,2,6,6-tetramethylpiperidin-1-oxybenzoate and the like can be mentioned.

The amount of the TEMPO catalyst to be used is generally 0.0001-0.3, preferably 0.005-0.02, in a molar ratio relative to a 2',3'-hydroxyl-protected nucleoside compound.

As hypochlorite, for example, sodium hypochlorite, calcium hypochlorite and the like can be mentioned. As hypobromite, for example, sodium hypobromite and the like can be mentioned. Sodium hypochlorite and sodium hypobromite are commercially available generally in the form of an aqueous solution, and calcium hypochlorite is commercially available generally in the form of a solid.

The amount of hypochlorite or hypobromite to be used is generally 1.9-3.0, preferably 2.0-2.3, in a molar ratio relative to a 2',3'-hydroxyl-protected nucleoside compound. When the amount is too small, the reaction becomes insufficient and the starting material tends to remain as an impurity. When the amount is too high, it is economically unpreferable, and the impurity due to hydrolysis tends to increase.

The pH for the oxidation is adjusted to the range of 5-9, preferably 6.5-8. When the pH is high, a 2',3'-hydroxyl-protected nucleoside compound tends to decompose and when the pH is too low, the reaction rate of the oxidation tends to decrease.

The oxidation can be carried out by, for example, adding hypochlorite or hypobromite to a solvent containing a 2',3'-hydroxyl-protected nucleoside compound and a TEMPO catalyst. The 2',3'-hydroxyl-protected nucleoside compound does not need to be completely dissolved in the solvent, and may be reacted in a suspension state as long as the object carboxylic acid compound is dissolved in a solvent in the system. While hypochlorite and hypobromite are preferably added in the form of an aqueous solution, in the case of, for example, commercially available solid such as calcium hypochlorite, it can be added as a solid. Addition of hypochlorite or hypobromite tends to increase pH, and when added in a short time, the pH value of the reaction mixture becomes too high to allow hydrolysis. Therefore, addition by small portions while adjusting pH to fall within the range of 5-9, preferably 6.5-8, is preferable. The addition can be performed generally in 30 min - 5 hr, preferably 2-4 hr. To facilitate adjustment of pH, the reaction may be carried out while dissolving a buffer such as sodium hydrogen carbonate, potassium hydrogen carbonate, sodium hydrogen phosphate and the like in a reaction mixture. The buffer may be dissolved in a reaction mixture from the start. Depending on the buffer, however, since the pH value tends to become high in the initial stage of the reaction, it is preferable to add an acid and the like where necessary to adjust the pH to fall within the above-mentioned optimal range and suppress the progress of hydrolysis as far as possible. Since production of the object carboxylic acid compound tends to lower the pH of the reaction mixture, even if the pH value is somewhat high in the initial stage of the reaction, it can be adjusted to the optimal range in a relatively short time by adding hypochlorite or hypobromite by small portions. The pH can be adjusted by controlling the addition rate of hypochlorite or hypobromite and buffer as mentioned above, as well as by appropriately adding a base such as sodium hydroxide, sodium carbonate, potassium hydroxide and the like, and an acid such as phosphoric acid, hydrochloric acid, sulfuric acid and the like. Even when the oxidation is conducted while adjusting pH, hydrolysis cannot be completely inhibited. However, the impurity generated by hydrolysis can be efficiently removed by obtaining the object crystal according to the crystal precipitation method of the present invention explained in the following.

After the completion of the reaction, it is preferable to decompose the oxidant (compound having an oxidizing ability derived from hypochlorite or hypobromite added) remaining in the reaction mixture by adding hydrogen sulfite such as sodium hydrogen sulfite and the like. Contamination of the object product with an oxidant possibly causes a problem of an adverse influence on the reaction during production of a derivative compound using the object product and the like. Hydrogen sulfite may be added in a solid state or an aqueous solution state. The amount of addition is not particularly limited, and it is preferable to confirm progress of the decomposition of the oxidant using a peroxide test paper (e.g., Merckoquant (trademark, manufactured by Merck)) and the like, and continue to add until the oxidant is completely decomposed. Use of sulfites such as sodium sulfite and the like is not preferable, because it is basic and tends to cause hydrolysis.

As a method of isolating a carboxylic acid compound as a solid from a reaction mixture, a known method comprising extraction with an organic solvent and concentration to dryness can be mentioned. However, this method shows poor purification efficiency and a highly pure object product cannot be obtained easily. In addition, a method comprising crystal precipitation using an organic solvent affords an oil and the like, and a crystal cannot be obtained easily, and the impurity caused by hydrolysis cannot be removed sufficiently. However, a crystal can be obtained stably by, after extraction of the object carboxylic acid compound into an organic solvent under acidic conditions, back-extracting the compound from the organic solvent into an aqueous alkali solution, and neutralizing the obtained aqueous alkali solution by adding an acid thereto to allow crystal precipitation of the carboxylic acid compound. In addition, highly pure carboxylic acid compound, from which impurity has been considerably removed, can be obtained.

The 2',3',5'-triacyloxynucleoside compound to be used as a starting material can be produced by, for example, a method described in Nucleic Acid Chem. (1991), 264-268. For example, 2',3',5'-triacetyl-6-chloropurineriboside, which is one of the starting materials, can be produced by adding dropwise thionyl chloride to 2',3',5'-triacetylinosine in a solvent, stirring under refluxing and workup by a conventional method.

The carboxylic acid compound of the present invention can be led to a compound such as adenosine A1 agonist etc. useful as a pharmaceutical product by, for example, a method described in JP-A-2000-514801 and the like or a method analogous thereto.

Because of the oxidation of only the 5'-position by the above-mentioned oxidation, the hydroxyl groups at the 2'-position and 3'-position of the nucleoside compound are preferably protected. As shown in the following reaction scheme, when the above-mentioned oxidation is applied to the oxidation of a nucleoside compound represented by the formula (1) defined below, a nucleoside-carboxylic acid compound represented by the formula (2) can be efficiently produced. In addition, hydrolysis that occurs during the oxidation can be also suppressed. 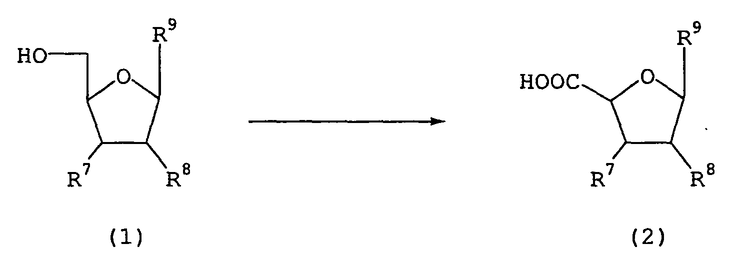 wherein each symbol in the formula is as defined below.

In the nucleoside compound represented by the formula (1), and a nucleoside-carboxylic acid compound represented by the formula (2), R⁹ shows a group of the following formula (3) or (4) :

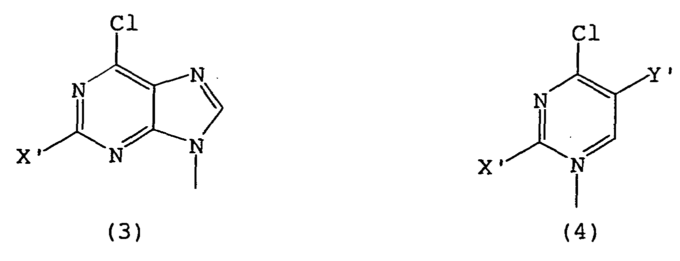

In other words, the nucleoside compound represented by the formula (1) shows a compound represented by the following (1-a) or (1-b), and a nucleoside-carboxylic acid compound represented by the formula (2) shows a compound represented by the following (2-a) or (2-b):

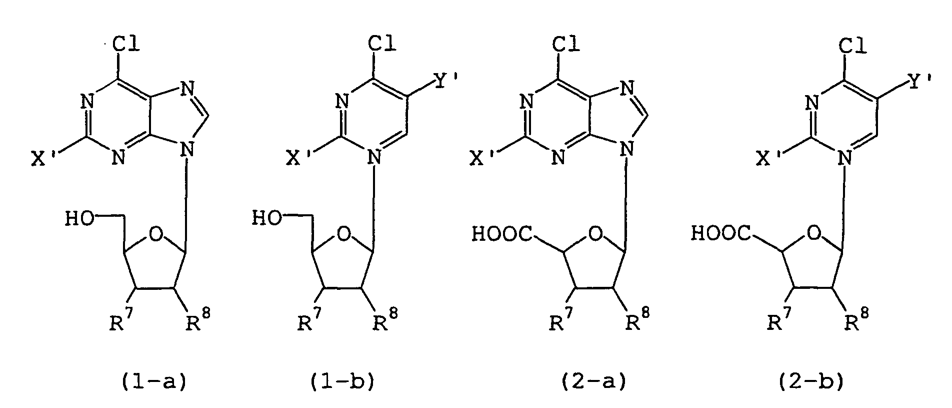

In the formulas in the present invention, X' is a hydrogen atom, a halogen atom, an amino group, a substituted (i.e., protected) amino group or a hydroxyl group.

As the halogen atom, a chlorine atom, a bromine atom, a fluorine atom and the like can be mentioned.

As the substituted (i.e., protected) amino group, for example, an acylamino group having 1 to 7 carbon atoms (e.g., acetylamino group, benzoylamino group etc.), an alkylamino group having 1 to 6 carbon atoms (e.g., methylamino group etc.), an aralkylamino group having 7 to 11 carbon atoms (e.g., benzylamino group) and the like can be mentioned.

In the formulas in the present invention, Y' is a hydrogen atom, a halogen atom, an alkyl group or an aralkyl group.

As the halogen atom, for example, chlorine atom, bromine atom, fluorine atom and the like can be mentioned.

As the alkyl group, for example, an alkyl group having 1 to 6 carbon atoms such as a methyl group and the like, and the like can be mentioned.

As the aralkyl group, for example, an aralkyl group having 7 to 11 carbon atoms such as a benzyl group and the like, and the like can be mentioned.

In the formulas in the present invention, R⁷ and R⁸ are each independently a hydrogen atom, an acyloxy group, an alkyloxy group, an aralkyloxy group or a *tert*-butyldimethylsilyloxy group, or R⁷ and R⁸ in combination show a group represented by the following formula (5):

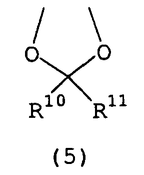

In the formula (5), R¹⁰ and R¹¹ are each independently an alkyl group. As the alkyl group, for example, an alkyl group having 1 to 6 carbon atoms such as a methyl group and the like, and the like can be mentioned.

As the acyloxy group, for example, an acyl group having 1 to 7 carbon atoms such as acetyloxy group, benzoyloxy group and the like can be mentioned.

As the alkyloxy group, for example, an alkyloxy group having 1 to 6 carbon atoms such as a methyloxy group and the like can be mentioned.

As the aralkyloxy group, for example, an aralkyloxy group having 7 to 11 carbon atoms such as a benzyloxy group and the like can be mentioned.

As the alkyl group represented by R¹⁰ or R¹¹, for example, an alkyl group having 1 to 6 carbon atoms such as a methyl group and the like can be mentioned.

The nucleoside compound represented by the formula (1) of the present invention can be produced according to a known method, for example, such as a method described in Journal of Organic Chemistry, 1987, vol. 52, pp. 1344-1347, a method described in Collection of Czechoslovak Chemical Communications, 2002, vol. 67, pp. 325-335 and the like.

In the present invention, a nucleoside-carboxylic acid compound represented by the formula (2) or a salt thereof can be obtained by oxidation (TEMPO catalyst oxidation) of the nucleoside compound represented by the formula (1) in the presence of a 2,2,6,6-tetramethylpiperidine-1-oxy catalyst (TEMPO catalyst), and hypochlorite or hypobromite, while adjusting pH to fall within the range of 5-9.

As a reaction solvent, a mixed solvent of water and an organic solvent, and a two-phase solvent wherein an aqueous phase and an organic solvent phase are phase-separated are preferable. The organic solvent may be any as long as it is free of an influence of an oxidation and, for example, for use for a mixed solvent with water, an organic solvent such as acetonitrile, tetrahydrofuran, acetone and the like can be mentioned, and for use for a two-phase solvent, an organic solvent such as chloroform, dichloromethane, tert-butylmethylether, acetates (e.g., methyl acetate, ethyl acetate etc.) and the like can be mentioned. The amount of the reaction solvent to be used is generally 3-50, preferably 5-20, in a weight ratio relative to a nucleoside compound (1). As mentioned above, when a mixed solvent of water and an organic solvent or a two-phase solvent is used, the whole solvent only needs to be included in this range.

As the 2,2,6,6-tetramethylpiperidine-1-oxy catalyst (TEMPO catalyst), 2,2,6,6-tetramethylpiperidine-1-oxy(TEMPO) and TEMPO-like compounds showing an oxidation catalytic function similar to that of TEMPO can be mentioned. As the TEMPO-like compound, for example, 4-hydroxy-2,2,6,6-tetramethylpiperidin-1-oxy, 4-methoxy-2,2,6,6-tetramethylpiperidin-1-oxy, 4-hydroxy-2,2,6,6-tetramethylpiperidin-1-oxybenzoate and the like can be mentioned.

The amount of the TEMPO catalyst to be used is generally 0.0001-0.3, preferably 0.005-0.02, in a molar ratio relative to nucleoside compound (1). As hypochlorite, for example, sodium hypochlorite, calcium hypochlorite and the like can be mentioned. As hypobromite, for example, sodium hypobromite and the like can be mentioned. Sodium hypochlorite and sodium hypobromite are commercially available generally in the form of an aqueous solution, and calcium hypochlorite is commercially available generally in the form of a solid.

The amount of hypochlorite or hypobromite to be used is generally 1.9-3.0, preferably 2.0-2.5, more preferably 2.0-2.3, in a molar ratio relative to nucleoside compound (1). When the amount is too small, the reaction becomes insufficient and the starting material tends to remain as an impurity. When the amount is too high, it is economically unpreferable, and the impurity due to hydrolysis tends to increase.

The pH for the oxidation is adjusted to the range of 5-9, preferably 6.5-8. When the pH is high, the nucleoside compound (1) tends to decompose and when the pH is too low, the reaction rate of the oxidation tends to decrease.

The oxidation can be carried out by, for example, adding hypochlorite or hypobromite to a solvent containing a nucleoside compound (1) and a TEMPO catalyst. The nucleoside compound (1) does not need to be completely dissolved in the solvent, and may be reacted in a suspension state as long as the object carboxylic acid compound is dissolved in a solvent in the system. While hypochlorite and hypobromite are preferably added in the form of an aqueous solution, in the case of, for example, commercially available solid such as calcium hypochlorite, it can be added as a solid. Addition of hypochlorite or hypobromite tends to increase pH, and when added in a short time, the pH value of the reaction mixture becomes too high to allow hydrolysis. Therefore, addition by small portions while adjusting pH to fall within the range of 5-9, preferably 6.5-8, is preferable. The addition can be performed generally in 30 min - 5 hr, preferably 2-4 hr. To facilitate adjustment of pH, the reaction may be carried out while dissolving a buffer such as sodium hydrogen carbonate, potassium hydrogen carbonate, sodium hydrogen phosphate and the like in a reaction mixture. The buffer may be dissolved in a reaction mixture from the start. Depending on the buffer, however, since the pH value tends to become high in the initial stage of the reaction, it is preferable to add an acid and the like where necessary to adjust the pH to fall within the above-mentioned optimal range and suppress the progress of the hydrolysis as far as possible. Since production of the object carboxylic acid nucleoside compound tends to lower the pH of the reaction mixture, even if the pH value is somewhat high in the initial stage of the reaction, it can be adjusted to the optimal range in a relatively short time by adding hypochlorite or hypobromite by small portions. The pH can be adjusted by controlling the addition rate of hypochlorite or hypobromite and buffer as mentioned above, as well as by appropriately adding a base such as sodium hydroxide, sodium carbonate, potassium hydroxide and the like, and an acid such as phosphoric acid, hydrochloric acid, sulfuric acid and the like. Even when oxidation is conducted while adjusting pH, hydrolysis cannot be completely inhibited. However, the impurity generated by hydrolysis during oxidation can be efficiently removed by obtaining the object crystal according to the crystal precipitation method of the present invention explained in the following.

After the completion of the oxidation, it is preferable to decompose the oxidant (compound having an oxidizing ability derived from hypochlorite or hypobromite added) remaining in the reaction mixture by adding hydrogen sulfite such as sodium hydrogen sulfite and the like. Contamination of the object product with an oxidant possibly causes a problem of an adverse influence on the reaction during production of a derivative compound using the object product and the like. Hydrogen sulfite may be added in a solid state or an aqueous solution state. The amount of addition is not particularly limited, and it is preferable to confirm progress of the decomposition of the oxidant using a peroxide test paper (e.g., Merckoquant (trademark, manufactured by Merck)) and the like, and continue to add until the oxidant is completely decomposed. Use of sulfites such as sodium sulfite and the like is not preferable, because it is basic and tends to cause hydrolysis.

As a method of isolating a nucleoside-carboxylic acid compound as a solid from a reaction mixture, a method comprising extraction with an organic solvent and concentration to dryness to give a solid, as in the above-mentioned known method, can be mentioned. However, this method shows poor purification efficiency and a highly pure object product cannot be obtained easily. In addition, as mentioned above, even a method comprising crystal precipitation using an organic solvent affords an oil, hence a crystal cannot be obtained easily, and the impurity caused by hydrolysis cannot be removed sufficiently. However, a crystal of the object nucleoside-carboxylic acid compound can be obtained stably by, after extraction of the object nucleoside-carboxylic acid compound into an organic solvent under acidic conditions, back-extracting the compound from the organic solvent into an aqueous alkali solution, and neutralizing the obtained aqueous alkali solution by adding an acid thereto to allow crystal precipitation of the nucleoside-carboxylic acid compound. In addition, highly pure nucleoside-carboxylic acid compound, from which impurity has been considerably removed, can be obtained. Here, a crystal of a salt of the nucleoside-carboxylic acid compound can be also obtained by precipitating a crystal after extraction of the object nucleoside-carboxylic acid compound into an organic solvent under acidic conditions and back-extracting the compound from the organic solvent into an aqueous alkali solution to allow neutralization, or back-extracting the compound from the organic solvent into water and neutralization with an aqueous alkali solution. As the salt of the nucleoside-carboxylic acid compound, alkali metal salts such as sodium salt, potassium salt and the like, and the like can be mentioned.

A case wherein crystal precipitation of a nucleoside-carboxylic acid compound is performed sequentially from an oxidation is taken as an example and explained. Recrystallization of a solid of a nucleoside-carboxylic acid compound containing impurity for purification can be conducted according to this method.

First, a chlororiboside carboxylic acid compound present in a reaction mixture is extracted into an organic solvent under acidic conditions. To the reaction mixture is added an acid such as phosphoric acid, hydrochloric acid, sulfuric acid and the like and the pH is adjusted to the range of 1.5-3.5, preferably 2.0-3.0, to acidify the solution. When a two-phase solvent wherein water and an organic solvent are phase-separated is used, an acid is added to the aqueous layer side to adjust pH to the above-mentioned range. As the organic solvent to be used for extraction, ethyl acetate, chloroform, *tert*-butylmethyl ether and the like can be mentioned, with particular preference given to ethyl acetate. When a highly water-soluble organic solvent was used for the reaction mixture, it is preferable to add the above-mentioned organic solvent to the reaction mixture for extraction. In this case, the organic solvent may be added before or after the pH adjustment. After pH adjustment, extraction is performed by a conventional method and the organic layer, into which the object product has been extracted, is separated. While the temperature of extraction is not particularly limited, it is generally in the range of 10-40°C. The amount of the organic solvent to be used for the extraction is generally 5-50, preferably 8-20, in weight ratio relative to the object product. Extraction may be performed multiple times where necessary.

Then, the object product is back-extracted from the organic solvent, into which the object product has been extracted, into the aqueous alkali solution. A base such as sodium hydroxide, potassium hydroxide, sodium hydrogen carbonate and the like is added to water, pH is adjusted to the range of 5-12, preferably 6-8, to give an aqueous alkali solution. Extraction is performed using the above-mentioned organic solvent and the aqueous alkali solution by a conventional method and, after back-extraction of the object product into the aqueous layer (aqueous alkali solution layer), the aqueous layer, into which the object product has been extracted, is separated. While the temperature of extraction is not particularly limited, it is generally in the range of 0-40°C. The amount of water to be used for the extraction is generally 5-50, preferably 8-20, in weight ratio relative to the object product. Extraction may be performed multiple times where necessary.

Then, an acid is added to the aqueous alkali solution, into which the object product has been extracted, to perform neutralization to allow crystal precipitation. It is also possible to perform neutralization to allow crystal precipitation after appropriately adding an organic solvent miscible with water, such as methanol, ethanol, acetone and the like, to the aqueous alkali solution. In this case, the amount of the organic solvent to be added is in the range of generally 1-50 in volume ratio relative to the aqueous alkali solution. As the acid to be used for the neutralization to allow crystal precipitation, phosphoric acid, hydrochloric acid, sulfuric acid and the like can be mentioned. The pH of neutralization to allow crystal precipitation is generally 1.5-3.5, preferably 2.0-3.0. The temperature of crystal precipitation is generally 0-80°C, preferably 10-40°C. The crystal precipitation is generally performed under stirring. The slurry obtained by crystal precipitation is subjected to solid-liquid separation by a conventional method such as filtration, centrifugation and the like to isolate a crystal. Where necessary, the crystal may be washed with water, alcohol and the like, and a drying step may be introduced according to a conventional method. The crystal obtained in this way becomes a highly pure crystal, wherein the hydrolysate difficult to be removed can be efficiently removed by the method of the present invention.

Of the nucleoside-carboxylic acid compounds represented by the formula (2), 2',3'-isopropylidene-6-chloropurineriboside - 5'-carboxylic acid represented by the following formula (6) is preferable in consideration of the usefulness of an adenosine receptor agonist as a synthetic intermediate and easiness of deprotection:

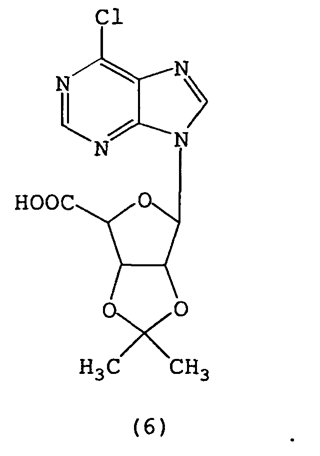

### Best Mode for Embodying the Invention

The present invention is explained in more detail in the following by referring to Examples, which are not to be construed as limitative.

### Preparation Example 1

### 2',3',5'-Triacetyl-6-chloropurineriboside

2',3',5'-Triacetylinosine (20 g) was added to chloroform (160 ml) and N,N-dimethylformamide (2.7 g), thionyl chloride (19.9 g) was added dropwise thereto, and the mixture was stirred under reflux for 3 hr. Water (200 ml) was added under cooling in an ice bath. The mixture was stirred for 1 hr and partitioned. The organic layer was washed with 5% aqueous sodium hydrogen carbonate solution and saturated brine, dried over sodium sulfate and concentrated to dryness to give 2',3',5'-triacetyl-6-chloropurineriboside (24.4 g) as an oil.
¹H-NMR(CDCl₃, ppm) δ: 2.10 (3H, s), 2.12(3H, s), 2.17 (3H, s), 4.37-4.51(3H, m), 5.64-5.67(1H, m), 5.94-5.97(1H, m), 6.24-6.25(1H, d, J=5.2Hz), 8.30(1H, s), 8.79(1H, s).

### Example 1

### 6-Chloropurineriboside

2',3',5'-Triacetyl-6-chloropurineriboside (oil, 6.0 g) was dissolved in methanol (30 ml). The mixture was cooled to 5°C and 1N sodium hydroxide-methanol solution (0.6 ml) was added. The mixture was stirred for 5 hr. Acetic acid (0.04 ml) and ethyl acetate (30 ml) was added to the reaction mixture and the mixture was stirred under ice-cooling for 1 hr. The precipitate was collected by filtration, washed with ethyl acetate, and vacuum dried at 40°C to give the title compound (3.08 g).
¹H-NMR(DMSO-d₆, ppm) δ: 3.59-3.74 (2H, m), 4.00-4.01(1H, s), 4.19-4.21(1H, m), 4.59-4.62(1H, m), 5.10-5.12(1H, m), 5.27(1H, d, J=5.1Hz), 5.59(1H, d, J=5.8Hz), 6.06(1H, d, J=5.3Hz), 8.83(1H, s), 9.06(1H, s).

### Example 2

### 2',3'-Isopropylidene-6-chloropurineriboside

6-Chloropurineriboside (10.0 g) was suspended in acetone (70 ml), 2,2-dimethoxypropane (7.3 g) and *p*-toluenesulfonic acid monohydrate (3.3 g) was added thereto, and the mixture was stirred at 10°C for 3 hr. The reaction mixture was added to a solution of sodium hydrogen carbonate (1.8 g) and water (70 ml). The mixture was concentrated under reduced pressure, and stirred at 20°C for 3 hr. The precipitate was collected by filtration, washed with water, and dried overnight at 40°C under reduced pressure to give 2',3'-isopropylidene-6-chloropurineriboside (9.7 g).
¹H-NMR(DMSO-d₆, ppm) δ: 1.34 (3H, s), 1.55(3H, s), 3.50-3.58(2H, m), 4.30-4.32(1H, m), 4.97-4.99(1H, m), 5.09-5.11(1H, s), 5.41-5.43(1H, s), 6.28(1H, d, J=2.4Hz), 8.82(1H, s), 8.87(1H, s).

### Example 3

### 2',3'-Isopropylidene-6-chloropurineriboside-5'-carboxylic acid

2',3'-Isopropylidene-6-chloropurineriboside (605 g, 1.085 mol) was added to a mixed solvent of acetonitrile (3630 ml) and water (1025 ml). Sodium hydrogen carbonate (106 g) and 2,2,6,6-tetramethylpiperidin-1-oxy (TEMPO, 5.8 g) were added thereto. An aqueous sodium hypochlorite solution (effective chlorine concentration 11%, 3034 g) was added dropwise over 3.4 hr while stirring at 5°C, and the mixture was further stirred for 1 hr. While the pH immediately after the start of the reaction was 9.5, it dropped to not more than 9 within 10 min after dropwise addition of the aqueous sodium hypochlorite solution. Thereafter, the pH was adjusted to 7 to 8 and this pH value was maintained during the reaction. After the completion of the reaction, 20% aqueous sodium hydrogen sulfite solution (1650 g) was added, and the mixture was stirred for 1 hr. At this time point, complete decomposition of the oxidant was confirmed using a peroxide test paper (Merckoquant, trademark, manufactured by Merck). The content of the impurity in the reaction mixture was confirmed by HPLC and found to be 3%. Then, ethyl acetate (4880 ml) was added to the reaction mixture, and the aqueous layer was adjusted to pH 2.8 with 6N hydrochloric acid, whereby extraction was carried out at 25°C. The organic solvent layer was separated, ethyl acetate (1120 ml) was added to the residual aqueous layer, which was then extracted again. The organic solvent layer was separated and combined with the organic solvent layer obtained earlier. Water (5064 ml) was added to the organic solvent layer and, after the aqueous layer was adjusted to pH 6.7 with aqueous sodium hydroxide solution, the mixture was subjected to a back-extraction at 25°C. The aqueous layer containing the extracted object product was separated. The aqueous layer was adjusted to pH 2.8 with 6N hydrochloric acid and the mixture was subjected to neutralization to allow crystal precipitation at 30°C. After crystal precipitation with stirring for about 17 hr, the slurry was filtered. The separated crystals were washed with water and dried overnight at 50°C under reduced pressure to give 2',3'-isopropylidene-6-chloropurineriboside-5'-carboxylic acid as crystals (493 g, 1.45 mol).
¹H-NMR(DMSO-d₆, ppm) δ: 1.37 (3H, s), 1.53 (3H, s), 4.79(1H, d, J=1.6Hz), 5.55(1H, dd, J=1.6, 5.9Hz), 5.61(1H, d, J=5.9Hz), 6.50(1H, s), 8.76(1H, s), 8.83(1H, s).

### Reference Example 1

### 2',3'-Isopropylidene-6-chloropurineriboside

6-Chloropurineriboside (640 g, 2.23 mol) was suspended in acetone (5120 ml), and dimethoxypropane (494 g) and p-toluenesulfonic acid monohydrate (212 g) were added. The mixture was stirred at 17-23°C for 5 hr. This reaction mixture was added to an aqueous solution of sodium hydrogen carbonate (99 g) and water (4480 ml). The aqueous solution was concentrated under reduced pressure, and stirred at 60°C for 2 hr and further at room temperature for about 17 hr. The obtained slurry was filtered, and the separated crystals were washed with water to give 2',3'-isopropylidene-6-chloropurineriboside as crystals (612 g, 1.87 mol).

### Example 4

### 2',3'-Isopropylidene-6-chloropurineriboside-5'-carboxylic acid

2',3'-Isopropylidene-6-chloropurineriboside (605 g, 1.85 mol) was added to a mixed solvent of acetonitrile (3630 ml) and water (3025 ml), and sodium hydrogen carbonate (106 g) and 2,2,6,6-tetramethylpiperidine-1-oxy (TEMPO) (5.8 g, 0.037 mol) were added. An aqueous sodium hypochlorite solution (effective chlorine concentration 11%, 3034 g, 4.25 mol) was added dropwise over 3.4 hr while stirring at 5°C, and the mixture was further stirred for 1 hr. While the pH immediately after the start of the reaction was 9.5, it dropped to not more than 9 within 10 min after dropwise addition of the aqueous sodium hypochlorite solution. Thereafter the pH was adjusted to 7 to 8 and this pH value was maintained during the reaction. After the completion of the reaction, 20% aqueous hydrogen sulfite sodium solution (1650 g) was added, and the mixture was stirred for 1 hr. At this time point, complete decomposition of the oxidant was confirmed using a peroxide test paper (Merckoquant, trademark, manufactured by Merck). The content of the impurity in the reaction mixture was confirmed by HPLC and found to be 3% (reaction yield was 95%). Then, ethyl acetate (4880 ml) was added to the reaction mixture, the aqueous layer was adjusted to pH 2.8 with 6N hydrochloric acid, whereby extraction was carried out at 25°C. The organic solvent layer was separated, ethyl acetate (1120 ml) was added to the residual aqueous layer, which was then extracted again. The organic solvent layer was separated and combined with the organic solvent layer obtained earlier. Water (5064 ml) was added to the organic solvent layer, and the aqueous layer was adjusted to pH 6.7 with aqueous sodium hydroxide solution. The mixture was subjected to a back-extraction at 25°C. The aqueous layer containing the extracted object product was separated, and the aqueous layer was adjusted to pH 2.8 with 6N hydrochloric acid. The mixture was subjected to neutralization to allow crystal precipitation at 30°C. After crystal precipitation with stirring for about 17 hr, the slurry was filtered. The separated crystals were washed with water and dried overnight at 50°C under reduced pressure to give 2',3'-isopropylidene-6-chloropurineriboside-5'-carboxylic acid as crystals (493 g, 1.45 mol). The content of the impurity in the crystals was confirmed by HPLC and found to be 0.1%.

### Example 5

### 2',3'-Isopropylidene-6-chloropurineriboside-5'-carboxylic acid

2',3'-Isopropylidene-6-chloropurineriboside (0.5 g, 1.53 mmol) was suspended in acetonitrile (3.5 ml) and water (3 ml), and sodium dihydrogen phosphate (0.4 g) was added to adjust its pH to 7. 2,2,6,6-Tetramethylpiperidin-1-oxy (TEMPO) (8 mg, 0.05 mmol) was added. An aqueous sodium hypochlorite solution (effective chlorine concentration 111%, 2.86 g, 3.87 mmol) was added dropwise over 60 min while stirring at 5°C, and the mixture was further stirred for 1 hr. During the reaction, the reaction mixture was maintained at pH 7.0-7.5. The reaction mixture was analyzed by HPLC. As a result, 2',3'-isopropylidene-6-chloropurineriboside-5'-carboxylic acid was produced in a yield of 92%, and the content of the impurity was 2%.

### Example 6

### 2',3'-Isopropylidene-6-chloropurineriboside-5'-carboxylic acid

2',3'-Isopropylidene-6-chloropurineriboside (0.6 g, 1.8 mmol) was suspended in acetonitrile (3.5 ml) and water (3 ml), and sodium hydrogen carbonate (0.2 g) and tetramethylpyridyl oxy (TEMPO) (8 mg, 0.05 mmol) were added. 60% Calcium hypochlorite (0.57 g, 4.39 mmol) was added in 4 portions over 1 hr while stirring at 5°C, and the mixture was further stirred for 1 hr. The pH immediately after the start of the reaction was 9.5, and it was adjusted to 7 to 8 in about 10 min after addition of the calcium hypochlorite. Thereafter, this pH value was maintained.

The reaction mixture was analyzed by HPLC. As a result, 2',3'-isopropylidene-6-chloropurineriboside-5'-carboxylic acid was produced in a yield of 91%, and the content of the impurity was 3%.

### Example 7

### Sodium 2',3'-isopropylidene-5'-carboxyl-6-chloropurineriboside-5'-acetate

2',3'-Isopropylidene-6-chloropurineriboside (6.05 g, 18.5 mmol) was added to acetonitrile (36 ml) and water (21 ml), and sodium hydrogen carbonate (0.4 g) and tetramethylpyridyloxy (TEMPO) (0.058 g, 0.37 mmol) were added. The mixture was stirred at 5°C. An aqueous sodium chlorite solution (effective chlorine concentration 11%, 30.4 g, 41.1 mmol) was added over 3 hr, during which the pH was maintained at 6.5-7.5 by the addition of 20% aqueous sodium hydrogen carbonate solution. The mixture was further stirred overnight, and the pH was maintained at 6.5-7.5. Sequentially, 20% aqueous sulfite hydrogen sodium solution (5.1 g) was added, and the mixture was stirred for 1 hr. At this time point, complete decomposition of the oxidant was confirmed using a peroxide test paper (Merckoquant, trademark, manufactured by Merck). The content of the impurity in the reaction mixture was confirmed by HPLC and found to be 1.8% (reaction yield was 97%). Ethyl acetate (49 ml) was added to the reaction mixture, and the mixture was adjusted to pH 2.7 with 6N hydrochloric acid. After layer separation, the aqueous layer was extracted with ethyl acetate (11 ml). Water (30 ml) was added to the combined organic layer and the mixture was adjusted to pH 6.7 with aqueous sodium hydroxide solution. The layers were separated and the aqueous layer was concentrated. Toluene (30 ml) was added and the mixture was stirred overnight. The precipitate was collected by filtration, washed with toluene, and dried overnight at 50°C under reduced pressure to give sodium 2',3'-isopropylidene-5'-carboxyl-6-chloropurineriboside-5'-acetate (6.5 g, 15.7 mmol). The content of the impurity in the crystal was confirmed by HPLC and found to be 1.4%.
¹H-NMR(DMSO-d₆) δ (ppm): 1.32(3H, s), 1.54(3H, s), 4.43(1H, s), 5.08(1H, d, J=5.9Hz), 5.18(1H, d, J=5.9Hz), 6.30(1H, s), 8.77(1H, s), 9.51(1H, s).

### Comparative Example 1

2',3'-Isopropylidene-6-chloropurineriboside (0.5 g, 1.5 mmol) was suspended in acetonitrile (3.5 ml) and water (3 ml), and sodium hydrogen carbonate (0.35 g) and 2,2,6,6-tetramethylpiperidine-1-oxy (TEMPO) (8 mg, 0.05 mmol) were added. An aqueous sodium hypochlorite solution (effective chlorine concentration 11%, 2.86 g) was added dropwise over 10 min while stirring at 5°C, and the mixture was further stirred for 1 hr. During the reaction, the pH of the reaction mixture was between 8.0 and 12.0. The reaction mixture was analyzed by HPLC. As a result, 2',3'-isopropylidene-6-chloropurineriboside-5'-carboxylic acid was produced in a yield of 85%, and the content of the impurity was 9%.

### Industrial Applicability

According to the present invention, formation of byproducts can be suppressed and nucleoside compound [II] can be produced. Therefore, nucleoside derivatives (2',3'-hydroxyl-protected nucleoside compound [III], carboxylic acid compound [IV]) can be also produced utilizing the nucleoside compound [II].

According to the present invention, moreover, the aforementioned nucleoside-carboxylic acid compound represented by the formula (2) and a salt thereof can be produced by a method suitable for industrial production. Thus, according to the present invention, hypochlorite or hypobromite, which is highly safe as an oxidant and which can easily control the reaction, can be used and hydrolysis, which is a side reaction, can be strikingly suppressed, in a production process of nucleoside-carboxylic acid compound of the formula (2), which is a 5'-carboxyl group derivative, comprising oxidation of a 5'-hydroxyl group of the nucleoside compound of the above-mentioned formula (1). In addition, the resulting hydrolysate can be efficiently removed, and highly pure crystals of a nucleoside-carboxylic acid compound represented by the formula (2) and a salt thereof can be produced by a method suitable for industrial production.

This application is based on patent application Nos. 010373/2003, 122614/2003 and 169534/2003 filed in Japan, the contents of which are hereby incorporated by reference.

## Claims

1. A production method of a nucleoside compound represented by the formula [II]: 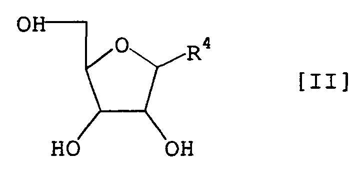 wherein R⁴ is a group represented by 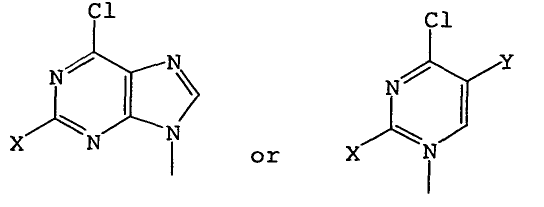 wherein X is a hydrogen atom, a halogen atom, an amino group, an alkyl group, an aralkyl group, a substituted amino group or a hydroxyl group, and Y is a hydrogen atom, a halogen atom, an alkyl group, an aralkyl group or an aryl group,
which comprises
subjecting a 2',3',5'-triacyloxynucleoside compound represented by the formula [I]: 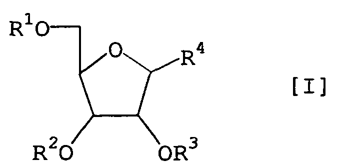 wherein R¹, R² and R³ are the same or different and each independently is an acyl group, and R⁴ is as defined above to deacylation using alkali metal hydroxide in a 0.01- to 0.5-fold amount in a molar ratio relative to the 2',3',5'-triacyloxynucleoside compound.

2. The production method of claim 1, wherein the deacylation is conducted in methanol, or a mixed solvent of methanol and an organic solvent.

3. The production method of claim 1 or 2, wherein the alkali metal hydroxide is sodium hydroxide.

4. A production method of a 2',3'-hydroxyl-protected nucleoside compound represented by the formula [III]: 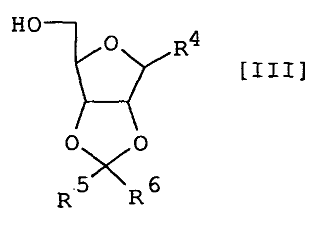 wherein R⁴ is as defined in claim 1, and R⁵ and R⁶ are optionally the same or different and each independently is an alkyl group, which comprises a step of obtaining a nucleoside compound represented by the formula [II] by the production method of any of claims 1 to 3.

5. A production method of a carboxylic acid compound represented by the formula [IV]: 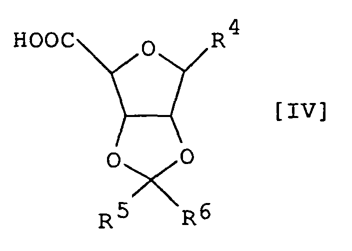 wherein R⁴ is as defined in claim 1, and R⁵ and R⁶ are optionally the same or different and each independently is an alkyl group, which comprises a step of obtaining a nucleoside compound represented by the formula [II] by the production method of any of claims 1 to 3.

6. The production method of claim 5, wherein the nucleoside compound of the formula [II] is converted to a 2',3'-hydroxyl-protected nucleoside compound of the formula [III], which is subsequently oxidized to give the carboxylic acid compound of the formula [IV].

7. A production method of a nucleoside-carboxylic acid compound represented by the formula (2) or a salt thereof, which comprises oxidizing a nucleoside compound represented by the formula (1) in the presence of a 2,2,6,6-tetramethylpiperidine-1-oxy catalyst, and hypochlorite or hypobromite, while adjusting pH to fall within the range of 5-9: 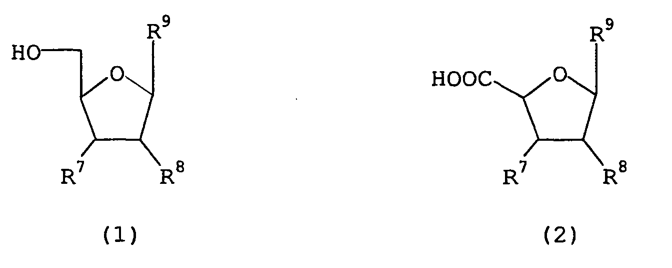 wherein R⁹ is a group represented by the following formula (3) or (4), and R⁷ and R⁸ are each independently a hydrogen atom, an acyloxy group, an alkyloxy group, an aralkyloxy group or a *tert*-butyldimethylsilyloxy group, or R⁷ and R⁸ in combination show a group of the following formula (5): 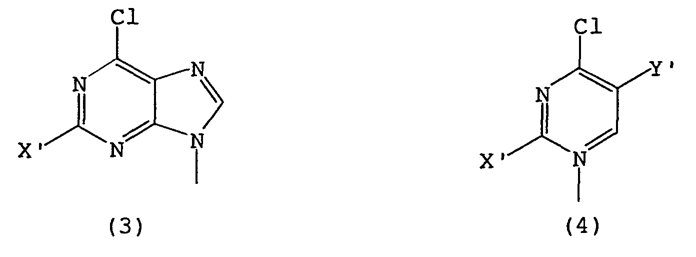 wherein X' is a hydrogen atom, a halogen atom, an amino group, a substituted amino group or a hydroxyl group, and Y' is a hydrogen atom, a halogen atom, an alkyl group or an aralkyl group: 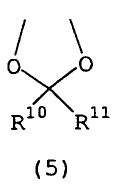 wherein R¹⁰ and R¹¹ are each independently an alkyl group.

8. The production method of claim 7, which comprises a step of decomposing the oxidant remaining in the reaction mixture with hydrogen sulfite after the completion of the oxidation.

9. The production method of claim 7, which comprises a step of producing a crystal of a nucleoside-carboxylic acid compound represented by the formula (2) by extracting the nucleoside-carboxylic acid compound in a reaction mixture into an organic solvent under acidic conditions, back-extracting the compound from the organic solvent into an aqueous alkali solution, and neutralizing the aqueous alkali solution by adding an acid thereto to allow precipitation of a crystal.

10. The production method of claim 7, which comprises a step of producing a crystal of a salt of the nucleoside-carboxylic acid compound represented by the formula (2) by precipitating a crystal after extracting the nucleoside-carboxylic acid compound in a reaction mixture into an organic solvent under acidic conditions, and back-extracting the compound from the organic solvent into an aqueous alkali solution to allow neutralization or back-extracting the compound from the organic solvent into water and neutralization with an aqueous alkali solution.

11. A production method of a crystal of a nucleoside-carboxylic acid compound represented by the formula (2), which comprises extracting the nucleoside-carboxylic acid compound represented by the formula (2) into an organic solvent under acidic conditions, back-extracting the compound from the organic solvent into an aqueous alkali solution, and neutralizing the aqueous alkali solution by adding an acid thereto to allow crystal precipitation of the nucleoside-carboxylic acid compound: 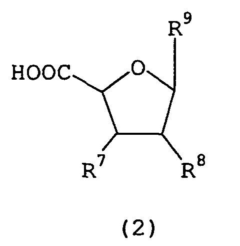 wherein R⁹ is a group represented by the following formula (3) or (4), and R⁷ and R⁸ are each independently a hydrogen atom, an acyloxy group, an alkyloxy group, an aralkyloxy group or a *tert*-butyldimethylsilyloxy group, or R⁷ and R⁸ in combination show a group represented by the following formula (5): 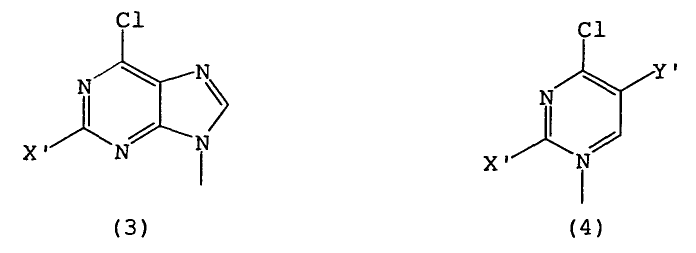 wherein X' is a hydrogen atom, a halogen atom, an amino group, a substituted amino group or a hydroxyl group, and Y' is a hydrogen atom, a halogen atom, an alkyl group or an aralkyl group, 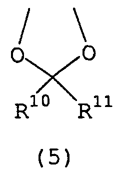 wherein R¹⁰ and R¹¹ are each independently an alkyl group.

12. The production method of any of claims 7 to 11, wherein the nucleoside-carboxylic acid compound represented by the formula (2) is a 2',3'-isopropylidene-6-chloropurineriboside-5'-carboxylic acid represented by the following formula (6):
